# EUROPÄISCHE PATENTANMELDUNG

(11) **EP 1 944 357 A1**
(43) Veröffentlichungstag der Anmeldung: **16.07.2008**
(21) Anmeldenummer: 06026722.6
(22) Anmeldetag: 22.12.2006
(51) Int. Cl.: C12M 1/28

(54) **Vorrichtung zur Durchführung eines Abklatschtests**

(71) Anmelder: Axon Lab AG, 5405 Baden-Dättwill (CH)
(72) Erfinder: Wiesner, Werner, D-94505 Bernried (DE); Müller, Guido, D-1715 Alterswil (CH)
(74) Vertreter: Gassner, Wolfgang

(57) **Zusammenfassung**

Die Erfindung betrifft eine Vorrichtung zur Durchführung eines Abklatschtests mit einem Behälter (1) und einem Deckel (2) zum Verschließen des Behälters (1), wobei an einem im verschlossenen Zustand vom Behälter (1) umschlossenen Raum hinweisenden Deckelboden (8) eine sich davon erstreckende Trägerplatte (12) zur Aufnahme eines Nährstoffmediums (18) angebracht ist. Insbesondere zur erleichterten Prüfung schwer zugänglicher Oberflächen wird erfindungsgemäß vorgeschlagen, dass die Trägerplatte (12) schwenkbar zwischen zwei mit dem Deckelboden (8) verbundenen Haltearmen (6) angebracht ist.

## Beschreibung

Die Erfindung betrifft eine vorrichtung nach dem Oberbegriff des Anspruchs 1.

Die Erfindung betrifft allgemein das Gebiet von Abklatschtests für die Hygienekontrolle. Sie betrifft insbesondere eine Vorrichtung zur Durchführung eines Abklatschtests mit einem Inkubator.

Eine derartige Vorrichtung wird beispielsweise unter der Marke "Hygicult" von der Firma AGA AG, CH-3285 Galmiz, angeboten. Die Vorrichtung umfasst einen Behälter und einen Deckel zum Verschließen des Behälters. An einem im verschlossenen zustand zum Behälterinnenraum hin weisenden Deckelboden ist eine sich davon erstreckende Trägerplatte angebracht, welche mit einem Nährstoffmedium für Bakterien beschichtet ist. Die Trägerplatte weist einen eingekerbten Abschnitt auf, um den sie entgegen einer zunehmenden elastischen Rückstellkraft geringfügig verbiegbar ist. Das geringfügige Verbiegen der Trägerplatte kann u. U. die Durchführung eines Abklatschtests an schwer zugänglichen Oberflächen erleichtern. - Nachteiligerweise wird bei einem Verbiegen der Trägerplatte das plastische Nährstoffmedium einer ungleichmäßigen und relativ hohen Druckkraft ausgesetzt. Das kann dazu führen, dass das Nährstoffmedium seitlich von der Trägerplatte weggedrückt wird oder an der zu testenden Oberfläche hängenbleibt. Ferner führt ein zu starkes verbiegen der Trägerplatte dazu, dass sie sich nicht mehr vollständig in die Ausgangsstellung zurückbewegt. Das behindert eine ordnungsgemäße Aufnahme der Trägerplatte im Behälter.

Aufgabe der Erfindung ist es, die Nachteile nach dem Stand der Technik zu beseitigen. Es soll insbesondere eine Vorrichtung zur Durchführung eines Abklatschtests angegeben werden, mit der auf einfache Weise auch eine Prüfung schwer zugänglicher Oberflächen möglich ist. Nach einem weiteren Ziel der Erfindung soll ein ungleichmäßiger und/oder zu hoher Anpressdruck auf das Nährstoffmedium vermieden werden.

Diese Aufgabe wird durch die Merkmale des Anspruchs 1 gelöst. Zweckmäßige Ausgestaltungen der Erfindung ergeben sich aus den Merkmalen der Ansprüche 2 bis 15.

Nach Maßgabe der Erfindung ist vorgesehen, dass die Trägerplatte schwenkbar zwischen zwei mit dem Deckelboden verbundenen Haltearmen angebracht ist. - Damit kann die Trägerplatte um eine durch die Haltearme verlaufende Schwenkachse in einem weiten Bereich verschwenkt werden, ohne dass sie durch eine elastische Rückstellkraft in Richtung einer ursprünglichen Ausgangsposition zurückbewegt wird. Infolgedessen kann ein auf der Trägerplatte aufgenommenes Nährstoffmedium bei der Durchführung des Abklatschtests mit einem gleichmäßigen Druck beaufschlagt werden. Ein unerwünschtes Verdrücken des Nährstoffmediums oder ein Loslösen/Anhaften desselben an der zu prüfenden Oberfläche wird vermieden. Mit der vorgeschlagenen Vorrichtung können auch bei schwer zugänglichen Oberflächen Abklatschtests unter regulären Testbedingungen durchgeführt werden.

Nach einer vorteilhaften Ausgestaltung umfasst die schwenkbare Halterung der Trägerplatte zwei seitlich davon in gegenüberliegender Anordnung sich erstreckende Zapfen, welche in dazu korrespondierende, in der Nähe der freien Enden der Haltearme vorgesehene Ausnehmungen eingreifen. Die Zapfen sind zweckmäßigerweise in den Ausnehmungen verrastet. Die vorgeschlagene Vorrichtung ist besonders stabil. Die Trägerplatte kann sich auch im verschwenkten Zustand nicht ohne Weiteres von den Haltearmen lösen. Abgesehen von dem vorgeschlagenen Zusammenwirken der Zapfen mit den an den Haltearmen vorgesehenen Ausnehmungen ist es selbstverständlich auch möglich, die schwenkbare Halterung der Trägerplatte anders auszuführen. Beispielsweise können auch Zapfen an den Haltearmen vorgesehen sein, welche in dazu korrespondierende Ausnehmungen an der Trägerplatte eingreifen. Es ist auch denkbar, eine, beispielsweise aus Metall hergestellte, Achse durch die Haltearme und eine in der Trägerplatte vorgesehene Bohrung zu stecken, so dass die Trägerplatte schwenkbar um die Achse gehalten ist.

Nach einer weiteren vorteilhaften Ausgestaltung sind die Haltearme Bestandteil eines gabelartigen Halteelements, welches am Deckelboden befestigt ist. Indem die Haltearme Bestandteil eines Halteelements sind, kann die Montage der Haltearme am Deckelboden vereinfacht werden. Dazu kann das Halteelement zur Befestigung am Deckelboden ein Befestigungselement aufweisen. Das Befestigungselement kann ein herkömmliches Befestigungselement, beispielsweise ein Gewindebolzen, eine selbstschneidende Schraube oder dgl. sein. Vorteilhafterweise ist das Befestigungselement ein Steckelement, welches in eine dazu korrespondierende Einstecköffnung am Deckelboden, vorzugsweise verrastbar, einsteckbar ist. Selbstverständlich kann das Halteelement auch auf andere Weise am Deckelboden befestigt werden. Beispielsweise können das Halteelement oder die Haltearme mit dem Deckelboden verschweißt oder verklebt werden. Auch andersartig ausgebildete Rastverbindungen zur Befestigung des Halteelements oder der Haltearme am Deckelboden sind möglich.

Nach einer weiteren, vorteilhaften Ausgestaltung ist in der Nähe des einen Endes der Trägerplatte ein zumindest mit einem der Haltearme zusammenwirkendes Rastmittel vorgesehen, so dass die Trägerplatte in einer bezüglich der Haltearme im Wesentlichen parallelen Position verrastbar ist. Damit kann ein unerwünschtes Verschwenken der Trägerplatte verhindert werden, insbesondere wenn sie im Behälter aufgenommen ist. Das vorgeschlagene Rastmittel kann unterschiedlich ausgebildet sein. Nach einer vorteilhaften Ausgestaltung können beispielsweise zwei in einander gegenüberliegender Anordnung seitlich an der Trägerplatte vorgesehene Vorsprünge vorgesehen sein, welche in dazu an den Haltearmen vorgesehene, korrespondierend ausgebildete weitere Ausnehmungen eingreifen. Die vorgeschlagene Rastverbindung ist in diesem Fall so ausgebildet, dass sie bei einem leichten Druck gegen das eine Ende der Trägerplatte lösbar ist, so dass die Trägerplatte verschwenkbar ist.

Die Trägerplatte ist zweckmäßigerweise um zumindest 200° und vorzugsweise um zumindest 300° um die Schwenkachse verschwenkbar. Um die Schwenkbewegung der Trägerplatte zu begrenzen ist vorgesehen, dass das eine Ende der Trägerplatte näher an der Schwenkachse liegt als das andere Ende. In diesem Fall wird die Schwenkbewegung der Trägerplatte durch den Deckel begrenzt. Ferner ist es auch möglich, dass die Trägerplatte an ihrem anderen Ende mit einer etwa mittig davon sich erstreckenden Nase versehen ist. Die Nase kann in einem solchen radialen Abstand bezüglich der Schwenkachse angeordnet sein, dass sie beim Verschwenken der Trägerplatte am Deckel anschlägt. Das Vorsehen der vorgeschlagenen Nase hat den vorteilhaften Effekt, dass beim Anschlagen der Trägerplatte am Deckel ein auf der Trägerplatte aufgenommenes Nährstoffmedium nicht mit dem Deckel oder daran anliegenden Fingern einer Testperson in Berührung kommt.

Nach einer weiteren Ausgestaltung ist die Trägerplatte einseitig, vorzugsweise beidseitig, mit dem Nährstoffmedium beschichtet. Das ermöglicht in vorteilhafter Weise die Durchführung zweier Abklatschtests. Dazu kann die Trägerplatte auf ihrer einen Seite mit einem ersten Nährstoffmedium und auf ihrer anderen Seite mit einem zweiten Nährstoffmedium beschichtet sein. Das ermöglicht z. B. einen Test auf zwei unterschiedliche Bakterienarten.

Nach einer weiteren Ausgestaltung weist der Deckel an seiner inneren Umfangsfläche ein Innengewinde und der Behälter an seinem Hals ein dazu korrespondierendes Außengewinde auf. Das ermöglicht einen einfachen und dichten Verschluss des Behälters mit dem Deckel.

Von herstellungstechnischem Vorteil ist es, dass die Trägerplatte in einstückiger Ausbildung mit den Zapfen und dem Rastmittel aus spritzgegossenem Kunststoff hergestellt ist. In ähnlicher Weise kann auch das Halteelement in einstückiger Ausbildung mit den Haltearmen aus spritzgegossenem Kunststoff hergestellt sein. Auch das Steckelement kann Bestandteil der einstückigen Ausbildung des Halteelements sein.

Nachfolgend wird ein Ausführungsbeispiel der Erfindung anhand der Zeichnung näher erläutert. Es zeigen:
- Fig. 1: eine perspektivische Darstellung des Behälters mit Deckel,
- Fig. 2: eine erste Querschnittsansicht gemäß Fig. 1,
- Fig. 3: eine zweite Querschnittsansicht gemäß Fig. 1,
- Fig. 4: eine Detailansicht gemäß der Position C in Fig. 2,
- Fig. 5: eine perspektivische Darstellung des Halteelements,
- Fig. 6: eine Draufsicht auf das Halteelement gemäß Fig. 5 und
- Fig. 7: eine Querschnittsansicht gemäß der Schnittlinie A-A in Fig. 6.

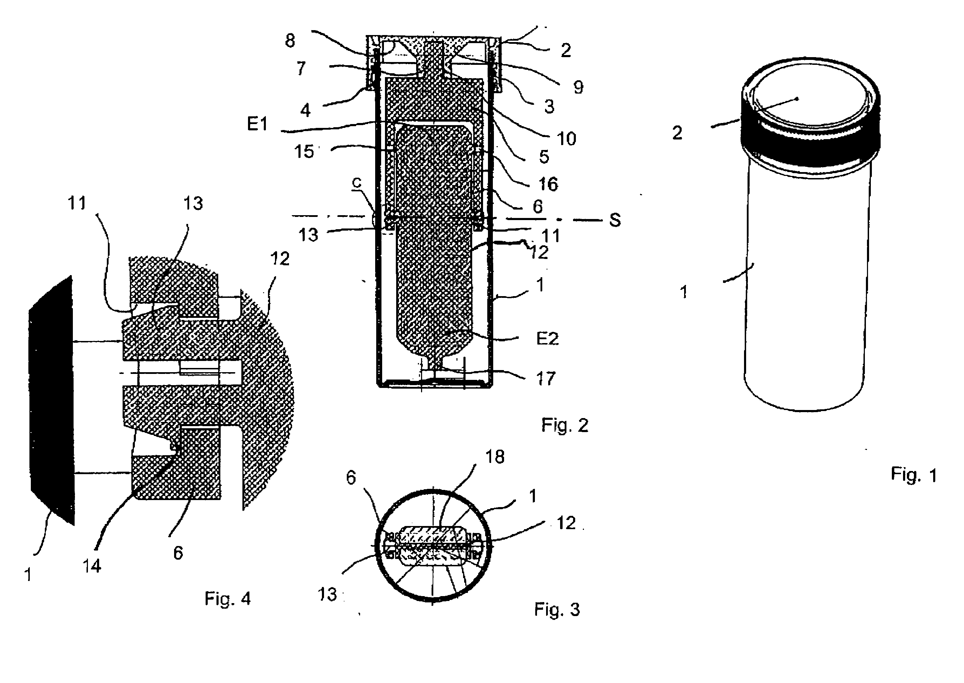

Die Fig. 1 und 2 zeigen einen zylindrischen, vorzugsweise aus einem transparenten Kunststoff hergestellten, Behälter 1, welcher mit einem Deckel 2 verschlossen ist.

Der Deckel 2 weist an seiner umlaufenden inneren Umfangsfläche ein Innengewinde 3 auf, das zur Ausbildung eines Schraubverschlusses mit einem am Hals des Behälters 1 vorgesehenen Außengewinde 4 korrespondiert.

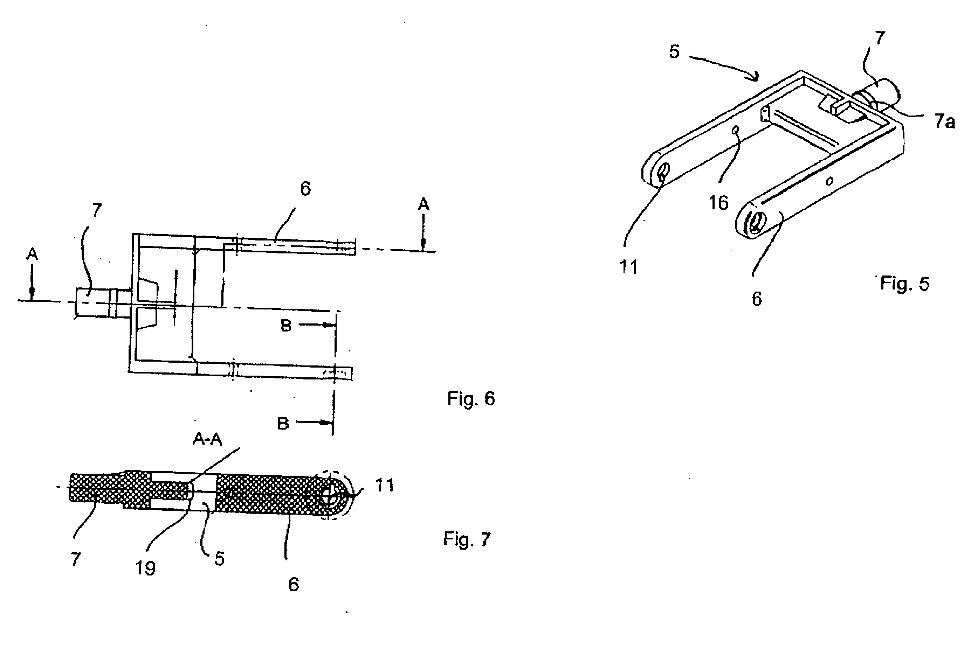

Ein gabelartig ausgebildetes Halteelement 5 weist zwei Haltearme 6 auf, welche sich in paralleler Anordnung in dieselbe Richtung erstrecken. Ferner erstreckt sich vom Halteelement 5 in eine zu den Haltearmen 6 entgegengesetzte Richtung ein Befestigungselement 7, welches hier nach Art eines ersten Zapfens ausgebildet ist und einen umlaufenden ersten Rastvorsprung 7a (siehe Fig. 5) aufweist. Ein zum Behälterinnenraum hin weisender Deckelboden 8 ist mit einer zentralen Erhebung 9 versehen, in der eine zum Befestigungselement 7 korrespondierende Einstecköffnung 10 vorgesehen ist.

Die Haltearme 6 weisen in der Nähe ihrer freien Enden erste Ausnehmungen 11 auf, in die von einer rechteckigen Trägerplatte 12 seitlich sich erstreckende Zapfen 13 eingreifen. Wie besonders gut aus Fig. 4 ersichtlich ist, sind die Zapfen 13 mit zweiten Rastvorsprüngen 14 versehen, so dass die Zapfen 13 drehbar in den ersten Ausnehmungen 11 gehalten, jedoch in axialer Richtung unverlierbar in den Haltearmen 6 aufgenommen sind.

Die Trägerplatte 12 weist in der Nähe ihres einen Endes E1 zwei seitlich davon sich erstreckende Vorsprünge 15 auf, welche in dazu korrespondierende, an den Haltearmen 6 vorgesehene zweite Ausnehmungen 16 zur Ausbildung einer lösbaren Rastverbindung eingreifen. In der Nähe eines anderen Endes E2 der Trägerplatte 12 ist eine Nase 17 angeformt.

Ein Abstand zwischen einer Schwenkachse S und dem ersten Ende E1 ist kleiner als ein Abstand zwischen dem zweiten Ende E2 und der Schwenkachse S. Die Trägerplatte 12 ist schwenkbar derart zwischen den Haltearmen 6 aufgenommen, dass sie mit der Nase 17 am Deckel 2 anschlägt.

Auf der Trägerplatte 12 ist - wie aus Fig. 3 ersichtlich ist - ein Nährstoffmedium 18, beispielsweise Agar aufgenommen. Die Trägerplatte 12 kann auf der einen Seite mit einem ersten Nährstoffmedium und auf der anderen Seite mit einem davon verschiedenen zweiten Nährstoffmedium beschichtet sein.

Fig. 5 zeigt nochmals in perspektivischer Ansicht das Halteelement 5 mit den Haltearmen 6 und dem Befestigungselement 7. Wie insbesondere in Zusammensicht mit den Fig. 5 und 6 ersichtlich ist, kann es sich bei dem Befestigungselement 7 um einen Zapfen handeln, der in eine am Halteelement 5 korrespondierende dritte Ausnehmung 18 eingesteckt ist. Das Befestigungselement 7 kann dort beispielsweise durch Kleben oder Verschweißen fixiert sein. Wie insbesondere aus den Fig. 5 und 7 ersichtlich ist, kann die in der Nähe der ersten Enden der Haltearme 6 vorgesehene Ausnehmung 11 auch nach Art eines Bajonettverschlusses ausgebildet sein. Das wirkt zusätzlich einem unerwünschten Lösen der Zapfen von den Haltearmen 6 entgegen.

Die vorgeschlagene Vorrichtung ermöglicht- ohne eine entgegenwirkende elastische Rückstellkraft - das Verschwenken der Trägerplatte um einen Winkel von zumindest 300° bezüglich der Haltearme 6. Die Trägerplatte 12 kann in einer im Wesentlichen parallel zu den Haltearmen 6 verlaufenden Richtung mittels der Vorsprünge 15 und der zweiten Ausnehmung 16 verrastet werden, so dass sie im, im Behälter 1 aufgenommenen, Transportzustand nicht mit der Behälterinnenwand in Berührung kommt. Die an der Trägerplatte 12 vorgesehene Nase 17 hat den besonderen Vorteil, dass damit eine Berührung des auf der Trägerplatte 12 aufgenommene Nährstoffmediums 18 mit dem Dekkel 2 oder den Deckel 2 haltenden Fingern einer Testperson vermieden werden kann.

### Bezugszeichenliste

- 1: Behälter
- 2: Deckel
- 3: Innengewinde
- 4: Außengewinde
- 5: Halteelement
- 6: Haltearm
- 7: Befestigungselement
- 7a: erster Rastvorsprung
- 8: Deckelboden
- 9: Erhebung
- 10: Einstecköffnung
- 11: erste Ausnehmung
- 12: Trägerplatte
- 13: Zapfen
- 14: zweite Rastvorsprünge
- 15: Vorsprünge
- 16: zweite Ausnehmung
- 17: Nase
- 18: Nährmedium
- 19: dritte Ausnehmung

- E1: Ende
- E2: anderes Ende
- S: Schwenkachse

## Patentansprüche

1. Vorrichtung zur Durchführung eines Abklatschtests, mit einem Behälter (1) und einem Deckel (2) zum Verschließen des Behälters (1), wobei an einem im verschlossenen Zustand zum Behälterinnenraum hin weisenden Deckelboden (8) eine davon sich erstreckende Trägerplatte (12) zur Aufnahme eines Nährstoffmediums (18) angebracht ist,
**dadurch gekennzeichnet, dass**
die Trägerplatte (12) schwenkbar zwischen zwei mit dem Dekkelboden (8) verbundenen Haltearmen (6) angebracht ist.

2. Vorrichtung nach Anspruch 1, wobei die schwenkbare Halterung der Trägerplatte (12) zwei seitlich davon in gegenüberliegenden Anordnung sich erstreckende zapfen (13) umfasst, welche in dazu korrespondierende, in der Nähe der freien Enden der Haltearme (6) vorgesehene Ausnehmungen (11) eingreifen.

3. Vorrichtung nach Anspruch 1 oder 2, wobei die Zapfen (13) in den Ausnehmungen (11) verrastet sind.

4. Vorrichtung nach einem der vorhergehenden Ansprüche, wobei die Haltearme (6) Bestandteil eines gabelartigen Halteelements (5) sind, welches am Deckelboden (8) befestigt ist.

5. Vorrichtung nach einem der vorhergehenden Ansprüche, wobei das Halteelement (5) zur Befestigung am Deckelboden (8) ein Befestigungselement (7) aufweist.

6. Vorrichtung nach einem der vorhergehenden Ansprüche, wobei das Befestigungselement (7) als Steckelement ausgebildet ist, welches in eine dazu korrespondierende Einstecköffnung (10) am Deckelboden (8), vorzugsweise verrastbar, einsteckbar ist.

7. Vorrichtung nach einem der vorhergehenden Ansprüche, wobei in der Nähe des einen Endes (E1) der Trägerplatte (12) ein zumindest mit einem der Haltearme (6) zusammenwirkendes Rastmittel (15, 16) vorgesehen ist, so dass die Trägerplatte (12) in einer bezüglich der Haltearme (6) im Wesentlichen parallelen Position verrastbar ist.

8. Vorrichtung nach einem der vorhergehenden Ansprüche, wobei die Trägerplatte (12) um zumindest 200°, vorzugsweise zumindest 300°, um eine durch die Haltearme (6) und die Trägerplatte (12) verlaufende Schwenkachse (S) verschwenkbar ist.

9. Vorrichtung nach einem der vorhergehenden Ansprüche, wobei das eine Ende (E1) der Trägerplatte (12) näher an der schwenkachse (S) liegt als das andere Ende (E2).

10. Vorrichtung nach einem der vorhergehenden Ansprüche, wobei die Trägerplatte (12) an ihrem anderen Ende (E2) mit einer etwa mittig davon sich erstreckenden Nase (17) versehen ist.

11. vorrichtung nach einem der vorhergehenden Ansprüche, wobei die Nase (17) in einem solchen radialen Abstand bezüglich der Schwenkachse (S) angeordnet ist, dass sie beim Verschwenken der Trägerplatte (12) am Deckel (2) anschlägt.

12. Vorrichtung nach einem der vorhergehenden Ansprüche, wobei die Trägerplatte (12) einseitig, vorzugsweise beidseitig, mit dem Nährstoffmedium (18) beschichtet ist.

13. Vorrichtung nach einem der vorhergehenden Ansprüche, wobei der Deckel (2) an seiner inneren Umfangsfläche ein Innengewinde (3) und der Behälter (1) an seinem Hals ein dazu korrespondierendes Außengewinde (4) aufweist.

14. Vorrichtung nach einem der vorhergehenden Ansprüche, wobei die Trägerplatte (12) in einstückiger Ausbildung mit den den Zapfen (13) und dem Rastmittel (15) aus spritzgegossenem Kunststoff hergestellt ist.

15. Vorrichtung nach einem der vorhergehenden Ansprüche, wobei das Halteelement (5) in einstückiger Ausbildung mit den Haltearmen (6) aus spritzgegossenem Kunststoff hergestellt ist.
